# EUROPÄISCHE PATENTSCHRIFT

(11) **EP 4 185 240 B1**
(45) Veröffentlichungstag und Bekanntmachung des Hinweises auf die Patenterteilung: **21.02.2024**
(21) Anmeldenummer: 21748809.7
(22) Anmeldetag: 12.07.2021
(51) Int. Cl.: A61F 2/16

(54) **INJEKTORANORDNUNG**
INJECTOR ARRANGEMENT
AGENCEMENT D'INJECTEUR

(30) Priorität: 21.07.2020 DE 102020119218
(43) Veröffentlichungstag der Anmeldung: 31.05.2023
(73) Patentinhaber: Carl Zeiss Meditec AG, 07745 Jena (DE)
(72) Erfinder: SCHREIBER, Benjamin, 10551 Berlin (DE); KELP, Martin, 12205 Berlin (DE); RINMAN, Alfred, 20357 Hamburg (DE)
(74) Vertreter: PATERIS Patentanwälte PartmbB
(86) Internationale Anmeldenummer: PCT/EP2021/069354
(87) Internationale Veröffentlichungsnummer: WO 2022/017855

(56) Entgegenhaltungen:
- DE-A1- 4 303 882
- US-B2- 10 695 166

## Beschreibung

Die Erfindung betrifft eine Injektoranordnung für ein Einführen einer Intraokularlinse in den Kapselsack eines Auges und betrifft einen Injektor mit der Injektoranordnung.

Bei der Kataraktbehandlung eines Auges wird herkömmlich nur ein kleiner Schnitt in die Hornhaut des Auges eingebracht, der so groß ist, dass eine Kanüle eines Injektors durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack eines Auges abgesaugt. Danach wird eine Intraokularlinse in das Auge eingesetzt. Dabei wird die Intraokularlinse gefaltet, so dass sie durch die Kanüle des Injektors passt. Die Kanüle wird durch den Schnitt in den Kapselsack eingeführt und die gefaltete Intraokularlinse wird mittels des Injektors durch die Kanüle in den Kapselsack geschoben, in dem die Intraokularlinse sich entfaltet und somit die ursprüngliche Linse ersetzt.

Ein Stempel des Injektors kann von einem die Behandlung durchführenden Arzt von Hand gedrückt werden. Es ist hierbei problematisch, dass ein Vorschub des Stempels ungleichmäßig erfolgen kann. Alternativ ist denkbar, dass der Arzt eine Antriebseinheit mit einem Motor verwendet, mittels der der Kolben verschoben wird. Es ist nachteilig, dass es sich bei der Antriebseinheit um eine Vorrichtung mit einem aufwändigen Aufbau handelt.

DE 43 03 882 A1 offenbart ein chirurgisches Kombinationsinstrument. US 10 695 166 B2 offenbart Intraokularlinsen.

Aufgabe der Erfindung ist es daher, eine Injektoranordnung mit einer Antriebseinheit und einen Injektor mit der Injektoranordnung zu schaffen, wobei die Antriebseinheit einen einfachen Aufbau hat.

Die vorliegende Erfindung bezieht sich auf eine Injektoranordnung nach Anspruch 1 und auf einen Injektor nach Anspruch 6. Bevorzugte Ausführungsweise sind in den Unteransprüchen definiert.

Bei dem Aktuator handelt es sich um ein Bauteil mit einem einfachen Aufbau. Die Erwärmungsvorrichtung kann ebenfalls mit einem einfachen Aufbau realisiert werden, beispielsweise indem die Erwärmungsvorrichtung von einer elektrischen Heizung geschaffen wird. Damit ist mittels des Aktuators und der Erwärmungsvorrichtung eine Antriebseinheit geschaffen, die einen einfachen Aufbau hat.

Es ist bevorzugt, dass der Aktuator eine Schraubenfeder aufweist oder eine Schraubenfeder ist. Die Schraubenfeder hat einen besonders einfachen Aufbau.

Zudem ist denkbar, dass der Aktuator, wenn er auf die Temperatur oberhalb der Übergangstemperatur erwärmt wird, seine Länge verkürzt oder seine Länge verlängert.

Es ist bevorzugt, dass der Formgedächtniswerkstoff eingerichtet ist, seine Phase von einer Martensitphase zu einer Austenitphase zu ändern, wenn der Aktuator auf die Temperatur oberhalb der Übergangstemperatur erwärmt wird. Dazu kann der Formgedächtniswerkstoff beispielsweise Eisen aufweisen. Besonders bevorzugt ist der Formgedächtniswerkstoff Nitinol.

Die Widerstandsmessvorrichtung ist bevorzugt eingerichtet, den elektrischen Widerstand von dem ersten Längsende bis zu dem zweiten Längsende zu messen. Dadurch, dass der Aktuator erwärmt wird, verlängert sich eine Länge des Aktuators. Unter der Annahme, dass bei dem Erwärmen ein Querschnitt des Aktuators unverändert bleibt, erhöht sich bei dem Erwärmen der elektrische Widerstand. Indem der Widerstand des Aktuators gemessen wird, ist es möglich zu bestimmen, wie weit der Aktuator seine Länge verlängert hat, um dadurch die tatsächliche Längsverschiebung der Intraokularlinse zu messen und über einen Heizstrom zu regeln. In dem Fall, dass der Aktuator die Schraubenfeder aufweist oder ist, ist die Länge die Länge eines Federdrahtes der Schraubenfeder und der Querschnitt ist der Querschnitt des Federdrahtes.

Es ist erfindungsgemäß, dass die Injektoranordnung eine Regelvorrichtung aufweist, die eingerichtet ist, die Temperatur des Aktuators unter Heranziehen des elektrischen Widerstands zu regeln. Dadurch ist es beispielsweise möglich, eine Geschwindigkeit einzustellen, mit der der Stempel längsverschoben wird. Auch ist es erfindungsgemäß, die Geschwindigkeit während des Längsverschiebens des Stempels zu erniedrigen. Ein Erniedrigen der Geschwindigkeit während des Längsverschiebens des Stempels wird erfindungsgemäß genutzt, damit die Geschwindigkeit des Stempels am Ende des Längsverschiebens niedrig ist. Dadurch kann verhindert werden, dass die Intraokularlinse mit einer hohen Geschwindigkeit und damit unkontrolliert einen die Injektoranordnung aufweisenden Injektor verlässt.

Die Erwärmungsvorrichtung weist bevorzugt eine Energiequelle auf, die ein Elektrizitätsspeicher und/oder ein chemischer Speicher ist. Der Elektrizitätsspeicher kann beispielsweise einen Akkumulator und/oder einen Kondensator aufweisen. Der chemische Speicher kann Wärme freisetzen, indem in dem chemischen Speicher eine exotherme Reaktion abläuft. Bei der exothermen Reaktion kann es sich beispielsweise um eine Oxidation von Eisen handeln. Alternativ ist denkbar, dass die Energiequelle einen physikalischen Speicher aufweist, der eingerichtet ist, beispielsweise eine Kristallisationswärme oder eine Adsorptionswärme freizusetzten.

Es ist bevorzugt, dass der Elektrizitätsspeicher eingerichtet ist, einen elektrischen Strom durch den Aktuator zu leiten. Dadurch kann der Aktuator unmittelbar erwärmt werden. Alternativ ist denkbar, dass die Erwärmungsvorrichtung einen Heizdraht aufweist und der Elektrizitätsspeicher eingerichtet ist, einen elektrischen Strom durch den Heizdraht zu leiten, wobei der Heizdraht neben dem Aktuator angeordnet ist und somit eingerichtet ist, den Aktuator zu erwärmen.

Der erfindungsgemäße Injektor weist die Injektoranordnung und eine Intraokularlinse auf, die in dem Injektor angeordnet ist.

Es ist bevorzugt, dass die Erwärmungsvorrichtung eingerichtet ist, die Intraokularlinse zu erwärmen. Dadurch wird die Intraokularlinse vorteilhaft flexibler und es wird somit einfacher, sie via eine Kanüle des Injektors aus dem Injektor herauszuschieben.

Es ist bevorzugt, dass der Injektor ein Injektorgehäuse aufweist, in dem die Energiequelle angeordnet ist. Es ist hier denkbar, dass der gesamte Injektor ein Einwegbauteil ist.

Es ist alternativ bevorzugt, dass der Injektor ein Injektorgehäuse aufweist und die Energiequelle außerhalb des Injektorgehäuses angeordnet ist. Es ist hier denkbar, dass der gesamte Injektor bis auf die Energiequelle ein Einwegbauteil ist und die Energiequelle wiederverwendbar ist.

Es ist alternativ bevorzugt, dass der Injektor eine Kartusche aufweist, in der die Intraokularlinse angeordnet ist und die lösbar mit der Injektoranordnung gekuppelt ist. Es ist hier denkbar, dass der Aktuator ein Einwegbauteil ist. Alternativ ist denkbar, dass der Aktuator nach einem Gebrauch wiederaufbereitet wird und erneut gebraucht wird. Es ist zudem denkbar, dass der Injektor ein Handteil aufweist, das ein von der Kartusche separates Bauteil ist und in dem der Aktuator angeordnet ist. Die Energiequelle kann außerhalb oder innerhalb des Handteils angeordnet sein.

Im Folgenden wird anhand der beigefügten schematischen Zeichnungen die Erfindung näher erläutert.
Figur 1 zeigt eine erste bevorzugte Ausführungsform eines Injektors, der eine erfindungsgemäße Injektoranordnung aufweist.
Figur 2 zeigt eine zweite bevorzugte Ausführungsform des Injektors.
Figur 3 zeigt eine Schraubenfeder zusammen mit einem Elektrizitätsspeicher.
Figur 4 illustriert eine Arbeitsweise der Schraubenfeder.

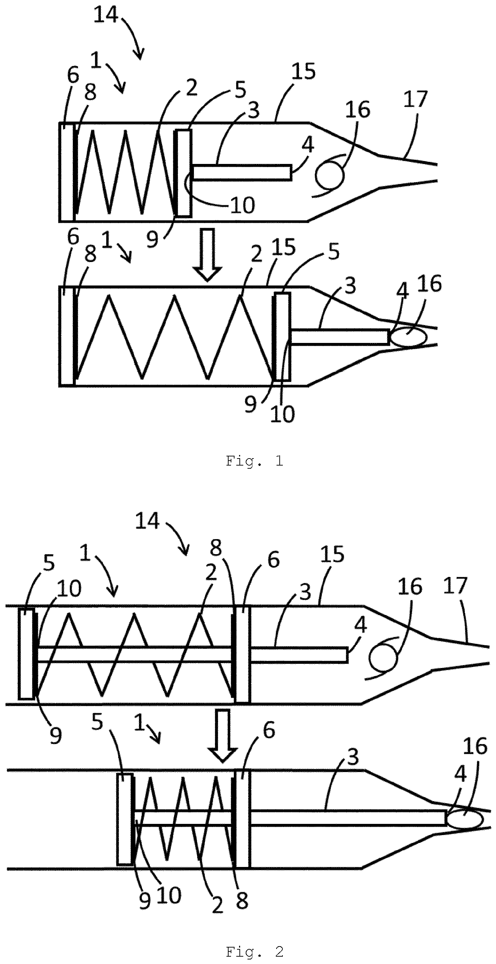

Wie es aus Figuren 1 bis 3 ersichtlich ist, weist eine Injektoranordnung 1 auf: einen Aktuator 2, der einen Formgedächtniswerkstoff mit einer Übergangstemperatur, ein erstes Längsende 8 und ein zweites Längsende 9 aufweist, einen Abstützsitz 6, an dem der Aktuator 2 im Bereich des ersten Längsendes 8 abgestützt ist, einen Stempel 3, der längsverschiebbar in der Injektoranordnung 1 gelagert ist, wobei der Stempel 3 eingerichtet ist, durch sein Längsverschieben eine Intraokularlinse 16 zu verschieben, und an dem zweiten Längsende 9 befestigt ist, und eine Erwärmungsvorrichtung, die eingerichtet ist, den Aktuator 2 auf eine Temperatur oberhalb der Übergangstemperatur zu erwärmen, wodurch der Aktuator 2 seine Länge verändert und somit der Stempel 3 längsverschoben wird. Dabei ist denkbar, dass der Aktuator 2 aus dem Formgedächtniswerkstoff besteht. Der Aktuator 2 kann beispielsweise eine Schraubenfeder 2 aufweisen oder eine Schraubenfeder sein.

Der Formgedächtniswerkstoff kann eingerichtet sein, seine Phase von einer Martensitphase zu einer Austenitphase zu ändern, wenn der Aktuator 2 auf die Temperatur oberhalb der Übergangstemperatur erwärmt wird. Dazu kann der Formgedächtniswerkstoff Eisen aufweisen. Zudem ist denkbar, dass der Formgedächtniswerkstoff Nitinol ist.

Figuren 1 und 2 zeigen jeweils eine bevorzugte Ausführungsform eines Injektors 14, der die Injektoranordnung 1 aufweist, wobei Figur 1 eine erste bevorzugte Ausführungsform zeigt und Figur 2 eine zweite bevorzugte Ausführungsform zeigt. Der Injektor 14 kann eine Intraokularlinse 16 aufweisen, die in dem Injektor 14 angeordnet ist. Zudem ist es denkbar, dass gemäß der ersten bevorzugten Ausführungsform und der zweiten bevorzugten Ausführungsform der Aktuator 2 eine Schraubenfeder 2 aufweist oder eine Schraubenfeder 2 ist.

Zudem zeigen Figuren 1 und 2, dass der Injektor 14 ein Injektorgehäuse 15 aufweisen kann, in dem der Aktuator 2 und der Stempel 3 angeordnet sind. Der Abstützsitz 6 kann an einer Innenseite des Injektorgehäuses 15 befestigt sein. Zudem ist denkbar, dass das erste Längsende 8 an dem Abstützsitz 6 befestigt ist. Aus Figuren 1 und 2 ist ersichtlich, dass die Injektoranordnung 1 eine Stempelbefestigung 5 aufweisen kann, die an dem zweiten Längsende 9 befestigt ist. Der Stempel 3 kann an der Stempelbefestigung 5 befestigt sein. Der Stempel 3 weist eine Stempelspitze 4 auf, die eingerichtet ist, die Intraokularlinse 16 zu kontaktieren, in eine Kanüle 17 des Injektors 14 und via die Kanüle 17 aus dem Injektor 14 heraus zu verschieben, wenn der Stempel 3 längsverschoben wird. Zudem weist der Stempel 3 ein der Stempelspitze 4 abgewandt angeordnetes Stempellängsende 10 auf, das an der Stempelbefestigung 5 befestigt sein kann.

Figuren 1 und 2 zeigen den Injektor 14 jeweils mit einer Temperatur des Aktuators 2 unterhalb der Übergangstemperatur und mit einer Temperatur des Aktuators 2 oberhalb der Übergangstemperatur, wobei das obere Bild jeweils den Zustand unterhalb der Übergangstemperatur zeigt. Bei der ersten bevorzugten Ausführungsform wird durch das Erwärmen des Aktuators 2 dessen Länge länger. Daher ist das erste Längsende 8 der Kanüle 17 abgewandt angeordnet und das zweite Längsende 9 ist der Kanüle 17 zugewandt angeordnet. Bei der zweiten bevorzugten Ausführungsform wird durch das Erwärmen des Aktuators 2 dessen Länge kürzer. Daher ist das erste Längsende 8 der Kanüle 17 zugewandt angeordnet und das zweite Längsende 9 ist der Kanüle 17 abgewandt angeordnet. Der Stempel 3 erstreckt sich im Inneren des Aktuators 2 und durch eine Öffnung des Abstützsitzes 6 hindurch.

Es ist denkbar, dass der Injektor 14 ein Injektorgehäuse 15 aufweist, in dem die Energiequelle angeordnet ist. Es ist hier denkbar, dass der gesamte Injektor ein Einwegbauteil ist. Alternativ ist denkbar, dass der Injektor 14 ein Injektorgehäuse 15 aufweist und die Energiequelle außerhalb des Injektorgehäuses 15 angeordnet ist. Es ist hier denkbar, dass der gesamte Injektor 14 bis auf die Energiequelle ein Einwegbauteil ist und die Energiequelle wiederverwendbar ist. Alternativ ist denkbar, dass der Injektor 14 eine Kartusche aufweist, in der die Intraokularlinse 16 angeordnet ist und die lösbar mit der Injektoranordnung 1 gekuppelt ist. Es ist hier denkbar, dass der Aktuator 2 ein Einwegbauteil ist. Alternativ ist denkbar, dass der Aktuator 2 nach einem Gebrauch wiederaufbereitet wird und erneut gebraucht wird. Es ist zudem denkbar, dass der Injektor 14 ein Handteil aufweist, das ein von der Kartusche separates Bauteil ist und in dem der Aktuator 2 angeordnet ist. Die Energiequelle kann außerhalb oder innerhalb des Handteils angeordnet sein.

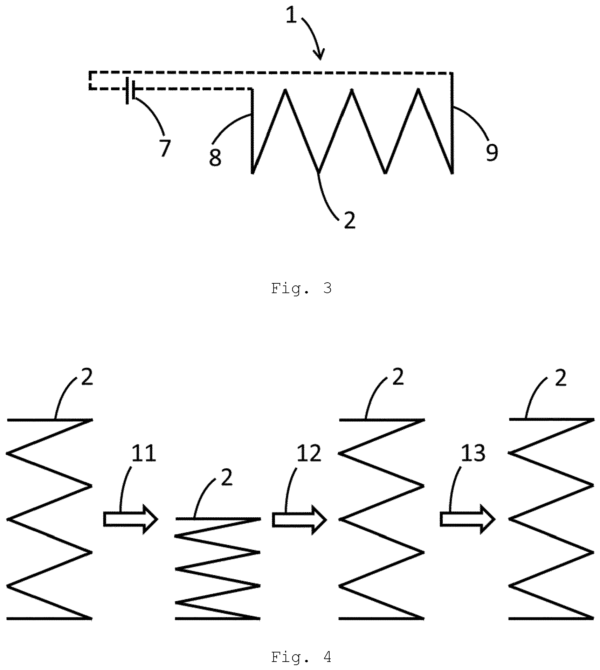

Die Erwärmungsvorrichtung kann eine Energiequelle aufweisen, die ein Elektrizitätsspeicher 7, ein chemischer Speicher oder ein physikalischer Speicher ist. Der chemische Speicher kann Wärme freisetzen, indem in dem chemischen Speicher eine exotherme Reaktion abläuft. Bei der exothermen Reaktion kann es sich beispielsweise um eine Oxidation von Eisen handeln. Der physikalische Speicher kann eingerichtet sein, eine Kristallisationswärme oder eine Adsorptionswärme freizusetzten. In Figur 3 ist dargestellt, dass der Elektrizitätsspeicher 7 eingerichtet sein kann, einen elektrischen Strom durch den Aktuator 2 zu leiten. Damit ist die Erwärmungsvorrichtung von dem Elektrizitätsspeicher 7 und dem Aktuator 2 gebildet. Einer der zwei Pole des Elektrizitätsspeichers 7 kann an das erste Längsende 8 und der andere der zwei Pole des Elektrizitätsspeichers 7 kann an das zweite Längsende 9 angeschlossen sein. Alternativ ist denkbar, dass die Erwärmungsvorrichtung einen Heizdraht aufweist und der Elektrizitätsspeicher 7 eingerichtet ist, einen elektrischen Strom durch den Heizdraht zu leiten, wobei der Heizdraht neben dem Aktuator 2 angeordnet ist und somit eingerichtet ist, den Aktuator 2 zu erwärmen.

Um die Intraokularlinse 16 flexibler zu machen, ist es denkbar, dass die Erwärmungsvorrichtung eingerichtet ist, die Intraokularlinse 16 zu erwärmen.

Die Injektoranordnung 1 weist eine Widerstandsmessvorrichtung auf, die eingerichtet ist, den elektrischen Widerstand des Aktuators 2 zu messen. Beispielsweise kann die Widerstandsmessvorrichtung an dem ersten Längsende 8 und an dem zweiten Längsende 9 angeordnet sein, so dass die Widerstandsmessvorrichtung eingerichtet ist, den elektrischen Widerstand des gesamten Aktuators 2 zu messen. Die Injektoranordnung 1 weist eine Regelvorrichtung auf, die eingerichtet ist, die Temperatur des Aktuators 2 unter Heranziehen des elektrischen Widerstands zu regeln.

In Figur 4 ist die Funktionsweise des Aktuators 2 beispielhaft anhand der Schraubenfeder 2 dargestellt. In dem linken Bild ist der Zustand der Aktuators 2 dargestellt, in dem er hergestellt ist. Wird der Aktuator 2 anschließend verformt, siehe Pfeil 11, wie beispielsweise verkürzt (wie in Figur 4 dargestellt) oder verlängert, so behält der Aktuator 2 diese Form. Wird Aktuator 2 anschließend über die Übergangstemperatur erwärmt, siehe Pfeil 12, so nimmt der Aktuator 2 die Form an, die er hatte, bevor er verformt 11 wurde. Wird der Aktuator 2 anschließend wieder unter die Übergangstemperatur abgekühlt, siehe Pfeil 13, so erfolgt keine weitere Formänderung.

### Bezugszeichenliste

1 Injektoranordnung
2 Aktuator, Schraubenfeder
3 Stempel
4 Stempelspitze
5 Stempelbefestigung
6 Abstützsitz
7 Elektrizitätsspeicher
8 erstes Längsende
9 zweites Längsende
10 Stempellängsende
11 Pfeil für die Zustandsänderung "Verformen"
12 Pfeil für die Zustandsänderung "Erwärmen"
13 Pfeil für die Zustandsänderung "Abkühlen"
14 Injektor
15 Injektorgehäuse
16 Intraokularlinse
17 Kanüle
18 Erwärmungsvorrichtung

## Patentansprüche

1. Injektoranordnung für ein Einführen einer Intraokularlinse in den Kapselsack eines Auges, mit einem Aktuator (2), der einen Formgedächtniswerkstoff mit einer Übergangstemperatur, ein erstes Längsende (8) und ein zweites Längsende (9) aufweist, einem Abstützsitz (6), an dem der Aktuator (2) im Bereich des ersten Längsendes (8) abgestützt ist, einem Stempel (3), der längsverschiebbar in der Injektoranordnung (1) gelagert ist, wobei der Stempel (3) eingerichtet ist, durch sein Längsverschieben eine Intraokularlinse (16) zu verschieben, und an dem zweiten Längsende (9) befestigt ist, und einer Erwärmungsvorrichtung, die eingerichtet ist, den Aktuator (2) auf eine Temperatur oberhalb der Übergangstemperatur zu erwärmen, wodurch der Aktuator (2) seine Länge verändert und somit der Stempel (3) längsverschoben wird, wobei die Injektoranordnung (1) eine Widerstandsmessvorrichtung aufweist, die eingerichtet ist, den elektrischen Widerstand des Aktuators (2) zu messen, wobei die Injektoranordnung (1) eine Regelvorrichtung aufweist, die eingerichtet ist, die Temperatur des Aktuators (2) unter Heranziehen des elektrischen Widerstands zu regeln, wobei die Regelvorrichtung eingerichtet ist, die Geschwindigkeit während des Längsverschiebens des Stempels (3) zu erniedrigen, damit die Geschwindigkeit des Stempels (3) am Ende des Längsverschiebens niedrig ist.

2. Injektoranordnung gemäß Anspruch 1, wobei der Formgedächtniswerkstoff eingerichtet ist, seine Phase von einer Martensitphase zu einer Austenitphase zu ändern, wenn der Aktuator (2) auf die Temperatur oberhalb der Übergangstemperatur erwärmt wird.

3. Injektoranordnung gemäß einem der Ansprüche 1 bis 2, wobei die Erwärmungsvorrichtung eine Energiequelle aufweist, die ein Elektrizitätsspeicher (7) und/oder ein chemischer Speicher ist.

4. Injektoranordnung gemäß Anspruch 3, wobei der Elektrizitätsspeicher (7) eingerichtet ist, einen elektrischen Strom durch den Aktuator (2) zu leiten.

5. Injektoranordnung gemäß einem der Ansprüche 1 bis 4, wobei der Aktuator (2) eine Schraubenfeder (2) aufweist oder eine Schraubenfeder (2) ist.

6. Injektor mit einer Injektoranordnung (1) gemäß einem der Ansprüche 1 bis 5, wobei der Injektor (14) die Intraokularlinse (16) aufweist, die in dem Injektor (14) angeordnet ist.

7. Injektor gemäß Anspruch 6, wobei die Erwärmungsvorrichtung eingerichtet ist, die Intraokularlinse (16) zu erwärmen.

## Claims

1. Injection arrangement for inserting an intraocular lens into the capsular sac of an eye, comprising an actuator (2) having a shape-memory material with a transition temperature, a first longitudinal end (8) and a second longitudinal end (9), a support seat (6) on which the actuator (2) is supported in the region of the first longitudinal end (8), a stamp (3) supported so as to be longitudinally displaceable in the injector arrangement (1), wherein the stamp (3) is configured to displace an intraocular lens (16) on account of itself being longitudinally displaced and is fastened at the second longitudinal end (9), and a heating apparatus configured to heat the actuator (2) to a temperature above the transition temperature, as a result of which the actuator (2) changes its length and the stamp (3) is thus longitudinally displaced, wherein the injector arrangement (1) comprises a resistance measurement apparatus configured to measure the electrical resistance of the actuator (2), wherein the injector arrangement (1) comprises a closed-loop control apparatus configured to control the temperature of the actuator (2) taking into account the electrical resistance, wherein the closed-loop control apparatus is configured to reduce the speed during the longitudinal displacement of the stamp (3) so that the speed of the stamp (3) is low at the end of the longitudinal displacement.

2. Injector arrangement according to Claim 1, wherein the shape-memory material is configured to change its phase from a martensite phase to an austenite phase when the actuator (2) is heated to the temperature above the transition temperature.

3. Injector arrangement according to either of Claims 1 and 2, wherein the heating apparatus comprises an energy source, which is an electricity storage unit (7) and/or a chemical storage unit.

4. Injector arrangement according to Claim 3, wherein the electricity storage unit (7) is configured to guide electrical power through the actuator (2).

5. Injector arrangement according to any of Claims 1 to 4, wherein the actuator (2) has a coil spring (2) or is a coil spring (2).

6. Injector having an injector arrangement (1) according to any of Claims 1 to 5, wherein the injector (14) comprises the intraocular lens (16) which is arranged in the injector (14).

7. Injector according to Claim 6, wherein the heating apparatus is configured to heat the intraocular lens (16) .

## Revendications

1. Ensemble injecteur destiné à insérer une lentille intraoculaire dans le sac capsulaire d'un oeil, ledit ensemble injecteur étant pourvu d'un actionneur (2) qui comporte un matériau à mémoire de forme ayant une température de transition, une première extrémité longitudinale (8) et une deuxième extrémité longitudinale (9), d'un siège d'appui (6) sur lequel l'actionneur (2) est en appui dans la zone de la première extrémité longitudinale (8), d'un piston (3) qui est monté de manière à pouvoir coulisser longitudinalement dans l'ensemble injecteur (1), le piston (3) étant conçu pour faire coulisser une lentille intraoculaire (16) en raison de son coulissement longitudinal et étant fixé à la deuxième extrémité longitudinale (9), et d'un dispositif de chauffage qui est conçu pour chauffer l'actionneur (2) à une température supérieure à la température de transition, ce qui amène l'actionneur (2) à changer de longueur et donc le piston (3) à coulisser longitudinalement, l'ensemble injecteur (1) comportant un dispositif de mesure de résistance qui est conçu pour mesurer la résistance électrique de l'actionneur (2), l'ensemble injecteur (1) comportant un dispositif de régulation qui est conçu pour réguler la température de l'actionneur (2) à l'aide de la résistance électrique, le dispositif de régulation étant conçu pour réduire la vitesse pendant le coulissement longitudinal du piston (3) de sorte que la vitesse du piston (3) soit faible à la fin du coulissement longitudinal.

2. Ensemble injecteur selon la revendication 1, le matériau à mémoire de forme étant conçu pour changer sa phase en passant d'une phase martensitique à une phase austénitique lorsque l'actionneur (2) est chauffé à la température supérieure à la température de transition.

3. Ensemble injecteur selon l'une des revendications 1 à 2, le dispositif de chauffage comportant une source d'énergie qui est un accumulateur d'électricité (7) et/ou un accumulateur chimique.

4. Ensemble injecteur selon la revendication 3, l'accumulateur d'électricité (7) étant conçu pour conduire un courant électrique à travers l'actionneur (2) .

5. Ensemble injecteur selon l'une des revendications 1 à 4, l'actionneur (2) comportant un ressort hélicoïdal (2) ou étant un ressort hélicoïdal (2).

6. Injecteur comprenant un ensemble injecteur (1) selon l'une des revendications 1 à 5, l'injecteur (14) comportant la lentille intraoculaire (16) qui est disposée dans l'injecteur (14).

7. Injecteur selon la revendication 6, le dispositif de chauffage étant conçu pour chauffer la lentille intraoculaire (16).
